# EUROPEAN PATENT SPECIFICATION

(11) **EP 4 371 517 B1**
(45) Date of publication and mention of the grant of the patent: **13.08.2025**
(21) Application number: 22208189.5
(22) Date of filing: 17.11.2022
(51) Int. Cl.: A61B 18/18

(54) **AN ABLATION PROBE HANDLE**
ABLATIONSSONDENGRIFF
MANCHE DE SONDE D'ABLATION

(43) Date of publication of application: 22.05.2024
(73) Proprietor: Endowave Ltd., D02 DK18 Dublin 2 (IE)
(72) Inventor: Ruvio, Giuseppe, Dublin 2, D02 DK18 (IE); Eaton-Evans, Jimmy, Dublin 2, D02 DK18 (IE); Hogan, Simon, Dublin 2, D02 DK18 (IE); Murray, Fergal, Dublin 2, D02 DK18 (IE); Bergin, Declan, Dublin 2, D02 DK18 (IE)
(74) Representative: Barker Brettell LLP

(56) References cited:
- US-A- 5 919 191
- US-A1- 2003 181 905

## Description

The present application relates to an ablation probe handle. Specifically, a handle for an ablation probe that suitable for use with an internal anatomy access device such as an endoscope. The ablation probe may be a microwave ablation probe.

Thermal ablation can be used to destroy tissue growths within the body which can be malignant. Current ablation systems use applicators that deliver Radio Frequency (RF) energy or more specifically microwave energy to the tissue surrounding an applicator tip. This causes localised heating and destruction of the malignant cells.

Microwave ablation systems generally comprise a microwave ablation probe which is used to deliver microwave ablation therapy to tissue. A microwave ablation probe may be used endoscopically so that it can be used to reach difficult to access ablation sites within the body, such as within the lungs. The ablation probe may therefore have a long catheter, containing a feed cable that supplies a microwave signal to an applicator or antenna at a distal end of the ablation probe. The catheter is inserted through the working channel of an endoscope or similar internal anatomy access device and then carefully positioned at the ablation site before the ablation therapy is activated.

At the proximal end of catheter, a handle is provided that allows the ablation probe to be manipulated by the user. The handle provides an interface where a working channel of an endoscope through which the ablation probe is inserted can be connected. The handle should allow the user to accurately control the position of the distal tip of the working channel, and the ablation probe being inserted through it, so that it can be positioned to deliver ablation at the required location. The handle also acts as an interface point at which the catheter is connected to a microwave signal generator and/or a coolant supply. For example, the handle may be connected using a lead cable to an ablation console where a microwave generator and a coolant pump to pump coolant to and from the distal tip of the probe are located.

Known handles for ablation probes have a number of drawbacks. The lead cable used to connect between the handle and ablation console is often thick and heavy. By connecting it to the handle the handle often becomes unwieldy and difficult to accurately control. The lead cable may have a levering effect on the handle and the connection to the working channel. This may make it difficult to maintain the position of the ablation probe while positioning it at the desired ablation site and difficult to keep stationary during ablation. These difficulties may result in the handle and lead cable needing a splint to support them during use, which is time consuming and can still be unstable.

In this context, inter alia, document US 2003/0181905 A1 forming a relevant piece of the art is mentioned.

It is also important for the handle to have a natural comfortable grip and allow ease of access to its controls during use. It should be easy to use by a pulmonologist or thoracic surgeon who are often trained to manipulate a scope with their non-dominant hand and a primary tool (e.g. ablation probe) with their dominant hand. Without the handle having a comfortable and natural grip it may be difficult to accurately position the working channel and the ablation probe at the desired ablation site and keep it in position during the ablation therapy.

It is desirable for an ablation probe handle to be usable with a number of different types of internal anatomy delivery systems, and to integrate electrical and cooling connections safely without negatively impacting the ablation procedure.

The present application aims to provide an ablation probe handle that overcomes one or more of the design challenges above.

The invention and its most advantageous embodiments are defined in the appended set of claims.

In a first aspect, the present application provides an ablation probe handle for use with an ablation probe, the ablation probe being suitable for use with an internal anatomy access system, the ablation probe handle comprising any one or more of the following features:
a housing adapted to be gripped by the user;
a lead cable connection interface adapted for connection to a lead cable for supplying a source of microwaves, the lead cable connection interface defining a lead cable connection axis;
an ablation probe mounting adapted to mount the catheter of an ablation probe within the housing; and
a working channel connection interface adapted for connection to a working channel of an internal anatomy access system, wherein the catheter of the ablation probe is arranged to extend from the housing along the working channel when the working channel is connected to the handle, the working channel connection interface defining a working channel connection axis,
wherein the lead cable and working channel connection axes are parallel and offset relative to each other, and
wherein the connection interfaces are arranged such that when the working channel and the lead cable are connected to the handle the working channel extends from the housing in the same direction relative to the housing as the lead cable.

By arranging the connection interfaces and their respective connection axes in this way the weight distribution of the lead cable and handle may be improved and configured to reduce the load on the working channel connection. The separation between the connection axes may create a pivot point about which the handle can more easily be manipulated and the ablation probe positioned more easily and accurately at the ablation site.

The ablation probe mounting may be adapted to secure the catheter of the ablation probe within the housing such that an exposed portion of the length of the catheter is formed outside of the housing. The exposed portion may be proximal along the length of the catheter relative to the point at which the catheter extends from the working channel connection interface. The catheter may be movable along the working channel by adjusting the length of the exposed portion outside of the housing.

The ablation probe mounting may be adapted to:
connect to a proximal end of the catheter of the ablation probe such that the proximal end is fixed relative to the housing; and
guide the path of the catheter such that the exposed portion of the length of the catheter is formed outside of the housing and a sliding portion of the catheter is arranged to pass through and slide with respect to the housing before passing through the working channel.

The exposed portion may be a loop of the catheter outside of the housing which may be moved into and out of the housing to adjust the position of the ablation probe along the working channel. The exposed part of the catheter may provide tactile feedback to the user to control the position of the ablation probe along the working channel.

The housing may comprise a first part and a second part. The lead cable connection interface may be provided on (or at) the first part of the housing. The ablation probe mounting may be adapted to secure the catheter of the ablation probe relative to the first part of the housing (e.g. it is connected to the lead cable within the first part of the housing) and slidably secure the catheter relative to the second part of the housing. The working channel connection interface may be provided on (or at) the second part of the housing. The first part of the housing may be slidable relative to the second part of the housing, whereby the catheter is slidable along the length the working channel. This may allow the user to move the ablation probe along the length of the working channel by moving the first and second parts of the housing relative to each other.

The handle may comprise a catheter locking mechanism. The catheter locking mechanism may be movable between an unlocked condition in which the catheter is slidable relative to the housing and an unlocked condition in which the catheter cannot slide relative to the housing. This may allow the user to fix the position of the ablation probe relative to the handle when it is in the desired position for ablation.

The handle may comprise a sliding mechanism. The sliding mechanism may comprise a sliding component on which the working channel connection interface is located. The sliding component may be slidable relative to the housing to allow independent movement of the working channel relative to the catheter.

The sliding mechanism may be used in combination with the catheter locking mechanism by locking the ablation probe in position relative to the handle and then using the sliding mechanism to move the working channel relative to the ablation probe while the ablation probe remains in a fixed position to un-sheath the distal end of the ablation probe from within the working channel before ablation is activated.

The housing may comprise an elongate grip portion adapted to be gripped single-handedly by the user. The grip portion may be shaped and configured to be gripped using a left of right handed grip.

The grip portion may have a non-circular cross-sectional shape. The non-circular profile may be an elongate shape having a major axis greater in length than a perpendicular minor axis. The cross-sectional shape may comprise two flat portions linked by two curved portions.

The handle may comprise one or more controls (e.g. actuators or other user activated buttons or input interfaces). The one or more controls may be spaced apart along the length of the housing from the grip portion. They may be spaced apart in a proximal direction along the length of the housing. The controls may be spaced from the grip portion such that they can be reached by the user's thumb when their fingers are wrapped around the grip portion. This may allow the user to operate the controls without repositioning their grip. The controls may include a control for the catheter locking mechanism used to fix the position of the catheter.

The handle may comprise one or more visual indicators. The visual indicator(s) may indicate a status of the ablation procedure. The visual indicators may, for example, be one or more coloured lights (e.g. LEDs) with different colours corresponding to a different status of the ablation procedure.

The grip portion may define a grip axis around which the housing is gripped by the user. The grip axis may be offset relative to the working channel connection axis. The grip axis may be a central longitudinal axis of the part of the housing gripped by the user. By offsetting the grip axis a pivot point may be created between the handle and the working channel. This may make the handle easier to manipulate when connected to the working channel compared to if the grip axis was in line with the working channel.

The working channel connection interface may be adapted to form a rotatable coupling with the working channel (e.g. between the working channel and the handle), whereby the handle is rotatable about the working channel connection axis during use. This may allow the user to rotate the handle about the axis of the working channel so that it is in a comfortable position while being used. This may also allow the user to adopt a left or right handed grip as required.

A portion of the length of the lead cable may extend along part of the length of the handle. The lead cable may extend within a portion of the housing, or alongside a portion of the housing. This may allow the lead cable to form a strong "spine" for the handle.

The housing may comprise a recessed portion in which the working channel or lead cable connection interface is located. This may provide space for a working channel to be connected (e.g. including any adaptors or other components at the proximal end of the working channel), and may allow use with a variety of different internal anatomy access systems.

The microwave ablation probe handle may further comprise a manifold adapted to connect to the lead cable and the catheter of the ablation probe. The manifold may be arranged to form an electrical connection between the lead cable and ablation probe when connected.

The lead cable may be arranged to carry a flow of coolant. The manifold may be arranged to form a fluidic connection between the catheter and lead cable when connected.

The manifold may provide a secure electrical and/or fluidic connection means between the lead cable and ablation probe within the handle without negatively impacting the ablation procedure.

The manifold may have a minimum fluidic and/or electrical connection distance within the handle.

The manifold may comprise an RF connector (such as a coaxial connector (for example MCX connector)) arranged to provide an electrical connection between a feed cable contained within the catheter of the ablation probe and the lead cable.

The manifold may comprise a coolant inflow channel arranged to provide a supply of coolant to the catheter of the ablation probe from the lead cable.

The manifold may comprise a coolant outflow channel arranged to carry a return flow of coolant from the catheter of the ablation probe to the lead cable. The manifold may be configured to minimise its length and therefore the length of feed cable contained within the catheter.

According to a second aspect, there is provided a handle system comprising the ablation probe handle of the first aspect. The handle system may further comprise a connector which is connectable between the lead cable and the working channel (when they are connected to the handle) at a point along the lead cable spaced apart from the housing. This may provide additional structure strength by using the strength of the lead cable.

According to a third aspect, there is provided an ablation system comprising the handle of the first aspect (or the handle system of the second aspect).

The ablation system may comprise an ablation probe mountable within the housing of the handle. The ablation probe may comprise a catheter having one or more transparent coolant conduits arranged to carry a flow of coolant. The coolant may be visible through the exposed portion of the catheter outside of the housing during use. This may allow the user to check that there are no air bubbles in the coolant. The microwave ablation system may further comprise the lead cable.

The catheter of the ablation probe may have a reference marker or markers arranged to indicate the position of the catheter within the working channel. The reference markers may comprise a distance scale arranged to indicate the length of the exposed portion of the catheter tube inserted into the handle. This may allow the user to accurately move the ablation probe into and out of the housing to adjust the position of the ablation probe along the working channel. The reference markers may additionally or alternatively indicate when the catheter has reached the distal end of the working channel, and/or when it is a specified distance from the distal end of the working channel. This may be particularly relevant to the ablation procedure.

The skilled person will appreciate that except where mutually exclusive, a feature described in relation to any one of the above aspects may be applied to any other aspect.

Embodiments of the invention will now be described, by way of example only, with reference to the accompanying drawings:
**Figure 1** shows a schematic illustration of a microwave ablation system being used with an endoscope;
**Figure 2a** shows a side view of an ablation probe handle of the ablation system of Figure 1;

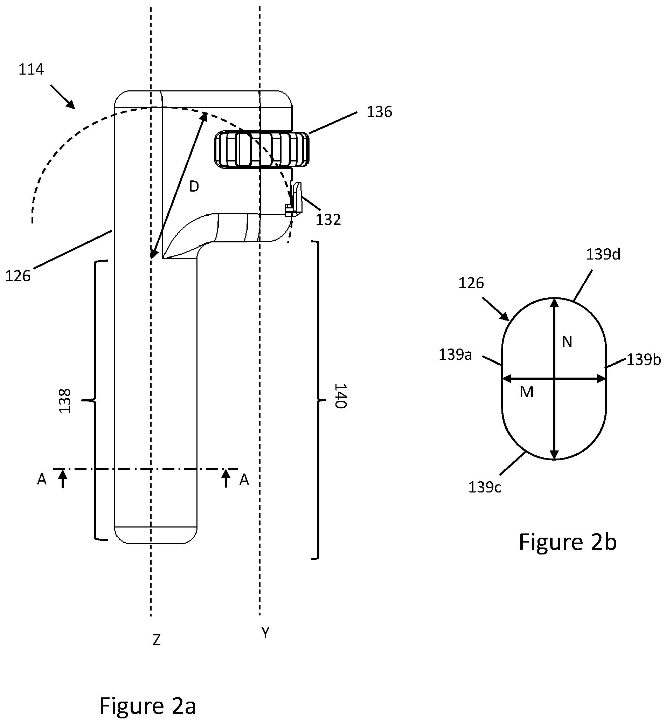

**Figures 2a to 2f** illustrate the shape of the cross-sectional profile of a grip portion of the handle;

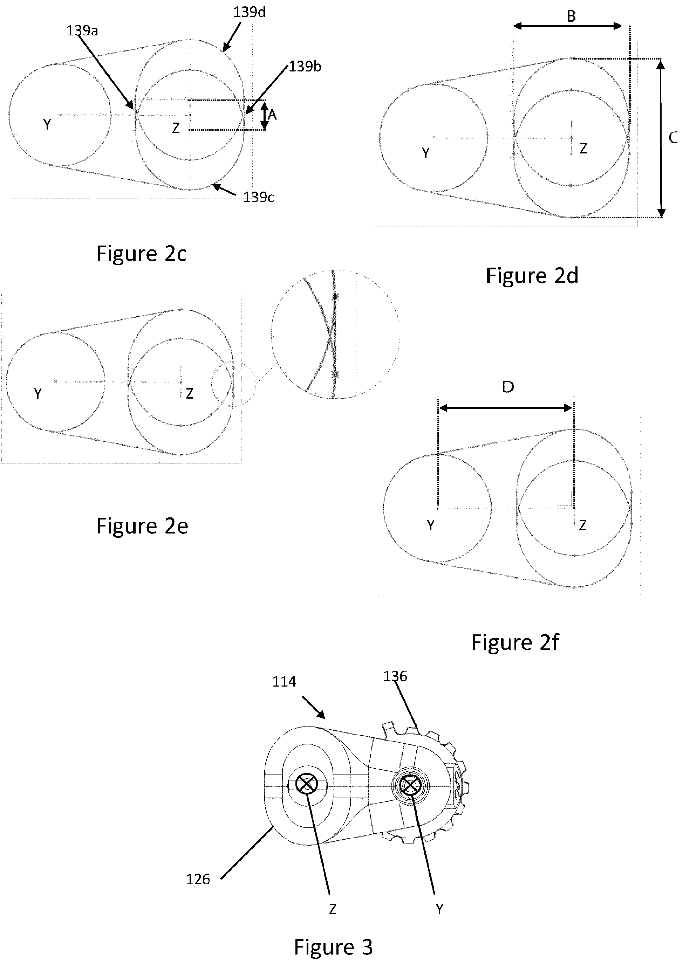

**Figure 3** shows an end view of the handle shown in Figure 2;

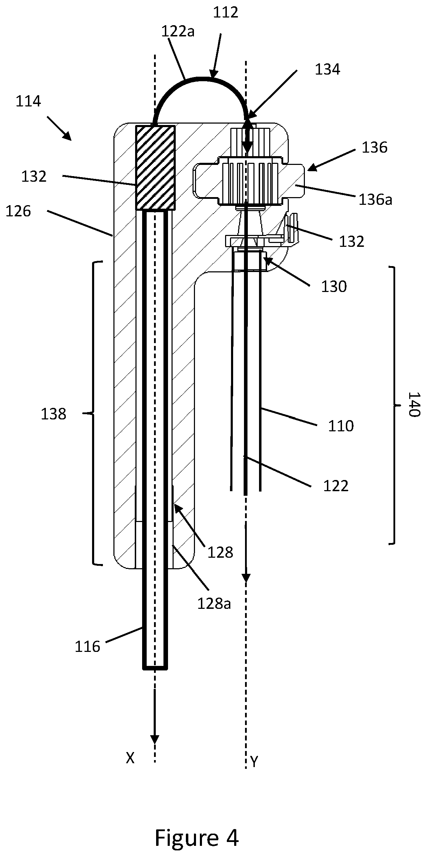

**Figure 4** shows a cross sectional view corresponding to the view of Figure 2a;

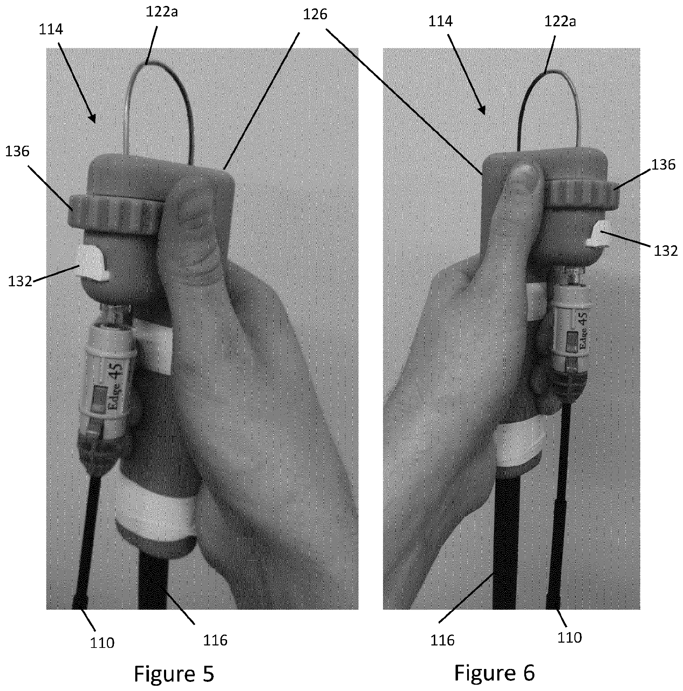

**Figures 5 and 6** show the handle of Figures 2a to 4 in use being held in a right and left handed grip respectively;

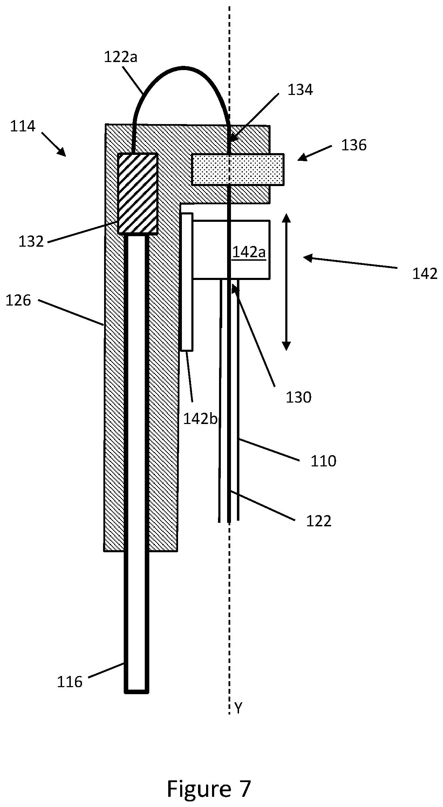

**Figure 7** shows a cross sectional view of an ablation probe handle according to another embodiment;

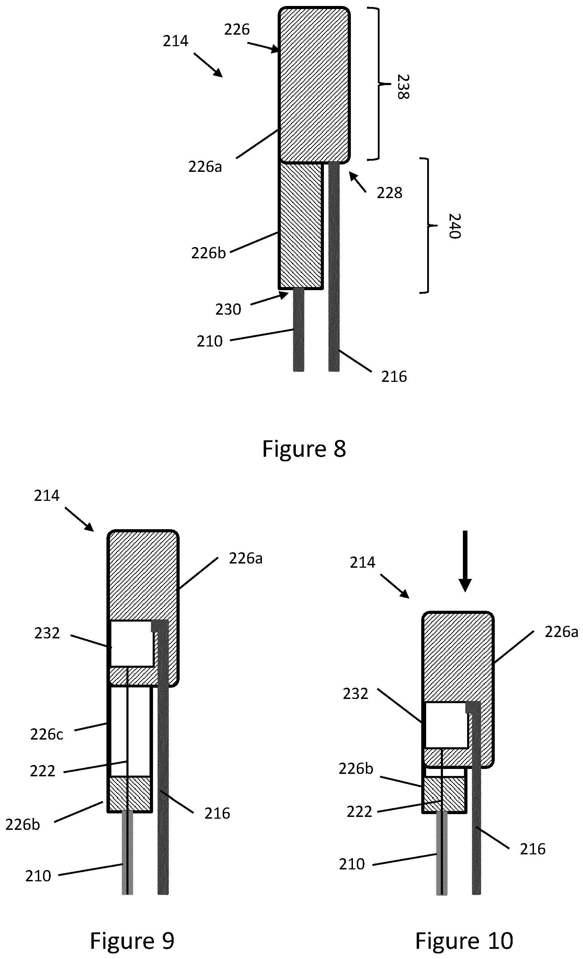

**Figure 8** shows a side view of an ablation probe handle according to another embodiment;
**Figures 9 and 10** show cross sectional views of the ablation probe handle of Figure 8 with a two-part housing in different configurations;

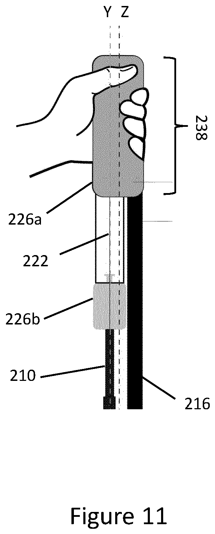

**Figure 11** shows the ablation probe handle of Figures 8 to 10 in use;

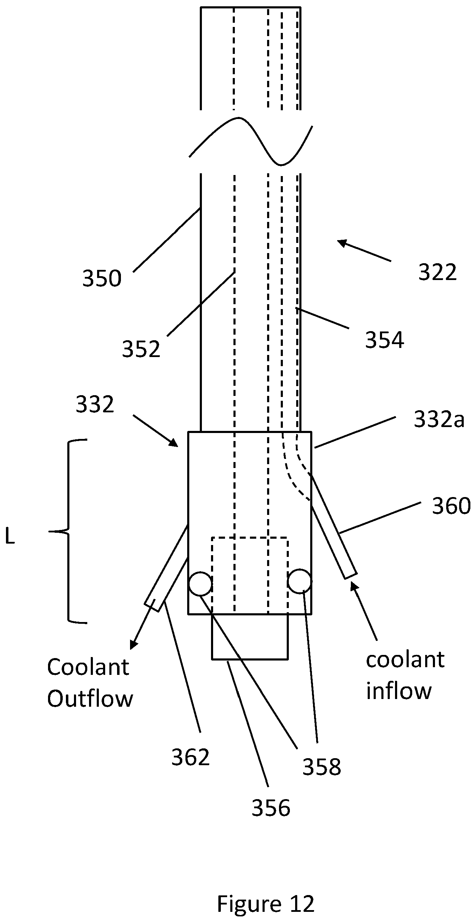

**Figure 12** shows a schematic view of a manifold which may form part of the handle of the present application; and

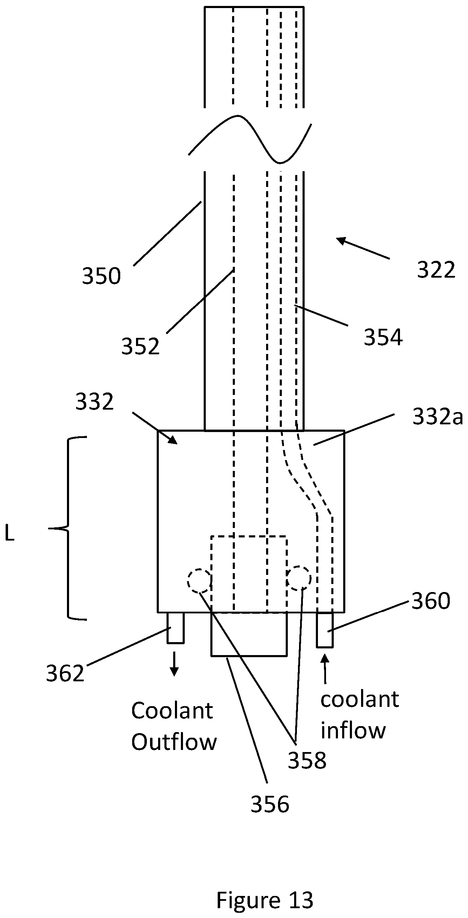

**Figure 13** shows an schematic view of another embodiment of the manifold of Figure 12.

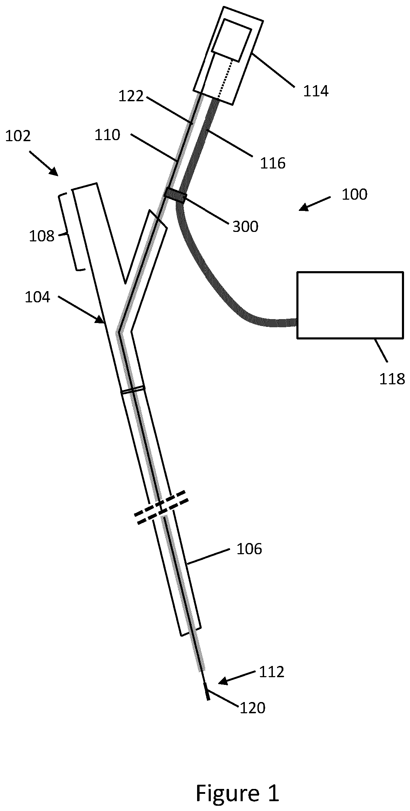

A microwave ablation system 100 being used with an internal anatomy access system 102 is shown schematically in Figure 1. The microwave ablation system 100 of the present disclosure is suitable for insertion into the body to reach a desired treatment site, such as a malignant tissue growth. In order to reach a desired treatment site, the ablation system includes an alation probe 112 suitable for insertion through a working channel of the internal anatomy access system 102 (which may be referred to as a delivery device or system). By internal anatomy access system we mean any device which may be placed within the anatomy of a patient, the device having a working channel for insertion of instruments to a desired location within the body. The internal anatomy access system may be an intraluminal delivery system arranged to be delivered along an anatomical lumen of the patient (e.g. the trachea and the pathways of the bronchi in the lungs or the oesophagus). The ablation system 100 may, for example, be used endoscopically or using an ENB (electromagnetic navigation bronchoscopy) system in order to reach a variety of disease locations within the body. The internal anatomy access system may comprise a bronchoscope, or other type of endoscope, or other type of internal navigation system required to reach any relevant part of the body.

In the presently described embodiment the delivery system 102 comprises an endoscope 104 having a main working channel 106 which can be inserted into the body of the patient as is known in the art. Only part of the length of the main working channel 106 is shown in Figure 1, but it may extend a suitable distance to access the desired location within the body, for example an ablation site within the lungs. The endoscope comprises a handle 108 with which it may be gripped by the user and manipulated. The delivery system 102 further comprises an extended working channel 110 which is inserted along the main working channel of the endoscope 104 to reach the ablation site. Various primary devices or tools such as an ablation probe can be inserted along the extended working channel 110.

The microwave ablation system 100 generally comprises a microwave ablation probe 112, a handle 114, a lead cable 116 and a console unit 118. The microwave ablation probe 112 comprises an applicator or antenna 120 located at its distal end via which microwave radiation is applied to surrounding tissue to cause localised heating and ablation. The ablation probe 112 further comprises a catheter 122 arranged to carry a microwave signal to reach the applicator. The catheter 112 may comprise a coaxial cable arranged to transmit the microwave signal within a catheter tube. The catheter is flexible such that it can be inserted and fed along the working channel(s) 106, 110 of the delivery system 102 to reach the ablation site. Again, only part of the length of the ablation probe is shown in Figure 1 to aid clarity. The handle of the present application can be used in conjunction with any suitable ablation probe that has a long flexible catheter for insertion along a working channel. The ablation probe may, for example, be as described in the applicant's earlier PCT application WO 2022/233938.

The term "catheter" refers generally to the elongate flexible part of the ablation probe which is inserted through the working channel. The catheter may comprise a feed cable adapted to transmit the microwave signal from the proximal end of the catheter to the applicator and may a cooling circuit through which coolant (for example water or saline) can be pumped to control the surface temperature of the catheter to a safe level (for example < 43 degs. C). The feed cable may be a coaxial cable. The catheter may comprise a catheter tube in which the feed cable is located.

The ablation probe extends between a proximal end and a distal end. The terms "distal" and "proximal" in the present application are taken relative to the user operating the ablation probe and the treatment site when the ablation probe is positioned for use - the distal end of the ablation probe or working channel(s) is that closest to the treatment site and the proximal end is that closest to the user. Any reference to a "length" herein is long an axis between distal and proximal points on a component.

The ablation probe 112 and the extended working channel 110 are connected to the handle 114 of the ablation system at their respective proximal end as will be described in more detail later. The handle 114 is adapted to be gripped by the user during use so that the extended working channel 110 and ablation probe 112 can be manipulated and positioned at the desired ablation site.

The handle 114 is also connected to the lead cable 116. The lead cable 116 is connected to the catheter 112 of the ablation probe within the handle 114 and is used to provide a supply of microwaves and/or coolant. The lead cable is connected to the console unit 118 which may act as a control unit at which the microwave ablation is controlled. The console unit 118 comprises a microwave generator which may be used to generate a microwave signal at the desired frequency. The microwave signal propagates from the console unit 118 to the handle 114 via the lead cable and then along the catheter 122 of the ablation probe 112 to the applicator 120 where microwave radiation is emitted. The console unit 118 may further supply a source of coolant used to cool the ablation probe 112, and in particular to cool the applicator 120. The ablation probe 112 may comprise one or more coolant channels (not shown in the figures) extending along the length of the catheter 122 which may form a coolant circuit arranged to carry coolant to the application 120 and back again. The console unit 118 may comprise a pump (not shown in the Figures) arranged to provide a flow of coolant to and from the catheter 122 via the lead cable. Although a single console unit 118 is shown in Figure 1 to provide both the microwave signal and coolant supply that arrangement may not be the case. The lead cable may only supply the microwave signal, or may include further branches to connect to a separate coolant supply system separate from the ablation console 118.

The lead cable may comprise a bundle of cables and/or other components, and may include any of: an RF feed cable, coolant tubes to provide a coolant circuit to the ablation catheter and/or low power electronic cables to provide power / communication to a PCB contained within the handle from the ablation console.

The ablation system 100 is shown in Figure 1 in an assembled condition as it would be during use, with the lead cable 116 and extended working channel 110 coupled to the handle 114, and the catheter 122 of the ablation probe 112 mounted in the handle 114 and extending through the extended working channel 110 and the endoscope main working channel 106. Some or all of these components may be provided separately and may be assembled with other suitable components to form the ablation system 100 shown. For example, the handle 114 may be provided separately. The handle 114 may be provided with the lead cable 116 ready attached or integral with it such that it can then be attached to a suitable console unit 118. The handle 114 may be provided separately from the extended working channel 122 (and other components of the delivery system 102), and can be used with a variety of different types of working channel as described below. The handle 114 can be provide separately from the ablation probe 112, which may be coupled to the handle 114 before use. A variety of different ablation probes can therefore be used with the handle of the present application.

Further details of the handle 114 are shown in Figures 2a to 6. Figure 2a shows a side view of the handle 114 in isolation without the other components of the ablation system 100. Figure 3 shows a corresponding end view. Figure 4 shows a cross sectional view corresponding to that of Figure 2a, with the lead cable 116, catheter 122 of the ablation probe 112 and extended working channel 110 present. Figures 4 and 5 show examples of the handle 114 in use.

The handle 114 comprises a housing 126. Part of the housing is adapted to be gripped by the user when using the handle 114. The handle 114 comprises a lead cable connection interface 128 and a working channel connection interface 130 at which the lead cable 116 and extended working channel 110 are connected, respectively.

The lead cable connection interface 128 in the present embodiment comprises a bore or hole 128a extending within the housing 126 into which the lead cable 116 is inserted and secured to a manifold 132. The manifold is located within the housing 126 of the handle 114, and is connected to the lead cable 116 and the feed cable 122 of the ablation probe. The manifold therefore provides a connection interface between the lead cable and ablation probe via which an electrical connection is provided for the propagation of microwaves and a fluidic connection is provided to supply the coolant circuit of the ablation probe. The handle therefore provides an integral electrical and coolant supply connection from the lead cable 116 to the ablation probe 112.

In other embodiments, other types of connection interface for the lead cable 116 may be provided. In some embodiments the manifold 132 may be absent, and a different connection between the ablation probe 112 and lead cable 116 provided by the handle 114. In the described embodiment the lead cable116 has a permanent fixed connection to the handle 114, however in other embodiments it may be removably connected.

A portion of the length of the lead cable 116 extends along part of the length of the handle 114. In the presently described embodiment, the lead cable 116 extends within the body of the housing 126 along part of the length of the housing 126 (e.g. along the bore 128a in the housing) before terminating at the manifold 132 (which is located near a proximal end of the handle). By extending along the length of the housing 126 in this way the lead cable 116 is used to form a "spine" of the handle 114. As the lead cable 116 is typically a relatively thick and heavy cable this provides improved strength for the handle 114 and may simplify the design. In other embodiments, the lead cable 116 may extend along part of the length of the handle to create a spine without being inside the housing 126 as will be described later. In yet other embodiments, the lead cable 116 may not extend within the body of the housing 126, but may connect at the outer wall of the housing 126.

The working channel connection interface 130 in the present embodiment comprises a luer connection and comprises a Luer lock 132 via which the extending working channel 110 of the delivery system may be connected and disconnected. The use of a luer connection may provide interoperability with a number of different prior art delivery systems. Other types of connection interface may be provided for the working channel 110. The working channel 110 may be removably connected via the working channel interface 130 to allow use with different delivery systems. The working channel connection interface may connect directly to the working channel as shown schematically in Figure 4, or may connect via an additional connecting component such as an adaptor or other interface component forming part of the delivery system 102.

The handle 114 further comprises a mounting 134 adapted to mount the ablation probe within the housing 126. As will be described in more detail later, the ablation probe (more specifically the catheter 122 of the ablation probe 112) extends through the housing such that it is mounted within the housing 126. The catheter 122 of the ablation probe 112 is arranged such that it extends from the outer wall of the housing at a point that coincides with the working channel connection interface 130. When the extended working channel 110 is connected to the handle 114 the path of the catheter 122 is thereby aligned with the working channel 110 and it can be inserted and fed along it. The catheter 122 may be slidably mounted with respect to the housing 126 (or part of the housing) such that it can be moved proximally or distally along the length of the working channel 110. This will be described in more detail later.

The lead cable connection interface 128 and the working channel connection interface 130 each define a respective connection axis i.e. a lead cable connection axis X and a working channel connection axis Y as shown in Figure 4. The lead cable connection axis extends through the point of connection of the lead cable 116, and defines an axis X along the direction in which the lead cable is connected or extends from a point on the outer surface of the housing 126 (e.g. the axis X extends normally to the surface of the housing at the respective connection interface). Similarly, the working channel connection axis extends through the point of connection of the extended working channel 110, and defines an axis Y along the direction in which the working channel is connected or extends from a point on the outer surface of the housing 126 (e.g. the axis Y extends normally to the surface of the housing at the respective connection interface). The connection interfaces 128, 130 are arranged on the handle 114 such that the lead cable and working channel connection axes X, Y are offset relative to each other as shown in Figure 4 and are or may also be parallel to each other. Moreover, the working channel may extend from the housing in the same direction relative to the housing as the lead cable as illustrated by the arrows in Figure 4. In other words, they both extend away from the housing 126 in a distal direction of the handle 114 (e.g. from a respective face of the housing on a distal side of the handle). Although the working channel and lead cable can be said to extend in the same direction away from the handle, it is to be understood that the lead cable brings power to the handle i.e. the direction of power flow is opposite to the direction which the lead cable extends from the handle shown by the arrow in Figure 4.

By arranging the connection interfaces 128, 130 as shown in Figure 4, the lead cable 116 and working channel 110 extend in the same direction away from the handle 114, and are offset from one another rather than connecting along the same longitudinal axis of the handle from its opposite ends. This connection arrangement helps to reconfigure the weight distribution of the lead cable 116 and handle 114 (e.g. compared to if they were inline), and may reduce or minimise the load on the delivery system (working channel) connection point. This may reduce the lever effect of the heavy lead cable 116 on the handle 114, which in turn may allow it to be more easily manipulated during use.

As discussed above, the handle comprises an ablation probe mounting 134 adapted to mount the catheter 112 of the ablation probe within the housing such that it extends along the length of the working channel 110. The ablation probe mounting is arranged to connect the ablation probe 112 at its proximal end to the lead cable 116 via the manifold 132. The proximal end of the catheter 122 is therefore anchored to a fixed point on or within the housing 126. The ablation probe mounting 134 is further arranged to allow the catheter 112 to slide through and relative to the housing 126 such that it can be inserted along the length of the working channel 110. The ablation probe mounting may comprise a guide hole through which the catheter 112 may pass so that it is slidably mounted in the housing. The ablation probe mounting 134 is arranged to form an exposed portion 122a of the catheter 122 which extends outside of the housing 126, after which the catheter further extends back into the housing such that it is directed along the connection axis Y of the working channel. The catheter is arranged to slide through the housing 126 as shown by the arrows in Figure 4. By adjusting the length of the exposed portion 122a the user may adjust the position of the catheter 122 of the ablation probe 112 within the working channel(s) 106, 110 and position the applicator 120 at the distal tip of the ablation probe 112 at the desired position for ablation.

As can be seen in Figure 4, the exposed portion 122a of the catheter forms a loop outside of the housing 126. This loop may be easily gripped and manipulated by the user. This is advantageous in allowing tactile feedback to the user. The action of pushing the catheter 122 in and out of the housing 126 via the exposed portion 122a allows the user to manipulate the ablation probe directly and may be an action that is similar to existing methods of feeding a device into the working channel of an endoscope. This may help the user operate the handle using already practiced skills.

In some embodiments, the catheter 112 may comprise one or more transparent coolant channels arranged to carry a flow of coolant along the length of the ablation probe 112 as described above. The exposed portion of the catheter 122a is further advantageous as it allows the coolant flow to be visible during use. For example, the user may be able to see any air bubbles that are within the cooling system within the exposed portion of the catheter which would require removable before ablation can occur.

In some embodiments, the catheter 112 of the ablation probe may have reference markers arranged to indicate the position of the catheter within the working channel. The reference markers may be provided on the part of the catheter forming the exposed portion 122a such that they are visible during use. The reference markers may comprise a distance scale arranged to indicate the length of the exposed portion of the catheter tube which has been inserted into the handle. This may indicate the length of the catheter which has been inserted into or pulled out of the handle, thus indicating how much the distal tip of the ablation probe is being moved. It may also include a maker or markers to indicate specifically when the distal tip of the catheter has reached the distal end of the working channel, and/or when the catheter has extended distally a specific distance from the tip of the working channel.

In the presently described embodiment, the handle 114 further comprises a catheter locking mechanism 136. The catheter locking mechanism 136 is movable between an unlocked condition in which the catheter 122 is slidable relative to the housing 126 and an unlocked condition in which the catheter 122 cannot slide relative to the housing 126. In the present embodiment, the locking mechanism comprises a rotary locking control. The rotary locking control comprises a rotating wheel 136a which may be operated by the user to lock and release the catheter 112. In the example described, the rotating wheel 136a may be mounted on a thread such that when rotated it moves along the length of the catheter 112 extending through it. The rotary locking control further comprises an elastomer material extending around the catheter which is compressed axially (e.g. in a direction along the length of the catheter) by the rotating wheel 136a when it is rotated. The elastomer material is constrained such that compression of the elastomer material in this way causes it to expand radially and grip the catheter 122 and resist it sliding through the housing. Rotation of the wheel in the opposite direction allows expansion of the elastomer material to release the catheter 122. This is to be understood as only one type of locking mechanism 136 that can be used to grip the catheter 112 and resist it sliding relative to the housing. The locking mechanism 136 may be used when the ablation probe has been positioned at the desired ablation site to fix it in position during ablation.

Referring again to Figures 2a and 4, the housing 126 comprises an elongate grip portion 138 adapted to be gripped by the user. The grip portion 138 is shaped such that it can be gripped single-handedly by the user as can be seen in Figures 5 and 6. The grip portion 138 is shaped such that when gripped by the user the user's arm has 0 to 20 degrees wrist extension and ulnar deviation. This may provide an optimum natural grip position. The grip portion 138 may be gripped by the user with one hand (e.g. their non-dominant hand) while the second hand is free to grip the handle 108 of the endoscope 104 or to adjust the position of the ablation probe.

The handle 114 comprises one or more controls that are arranged to control its operation. Such controls may include the locking mechanism 136 described above, but may include other controls to control the position of the ablation probe or other aspects of the device's operation. The grip portion 138 is arranged on the handle 114 with respect to the one more controls such that they are spaced apart from each other along the length of the housing 126. The controls may be spaced apart from the grip portion 138 in a proximal direction of the handle. The controls are positioned relative to the grip portion 138 such that they are operated by the user's thumb when the user's fingers are wrapped around the grip portion 138 without the user needing to change the position of their hand as can be seen in Figures 5 and 6. The controls may be spaced from the grip portion by a distance D which may be such that the user's thumb can reach them, and may for example be between 40 and 58 mm. The range of the reach of the user's thumb is shown by the dashed arc drawn in Figure 2a. By locating the controls in this position they may also be reached by both a left and right handed user as can be seen in Figures 5 and 6.

The handle may comprise one or more visual indicators. The visual indicator(s) may indicate a status of the ablation procedure. The visual indicators may for example, be one or more coloured lights (e.g. LEDs) with different colours corresponding to a different status of the ablation procedure. The visual indicators may be in communication with control circuitry provided at the console unit, and may indicate the ablation procedure status such as an indication the system is ready for ablation or ablation is activated, or an error has occurred. The visual indicators may allow the user to see the progress of the ablation procedure without having to turn and look at the console unit 118.

The visual indicators (coloured lights) may be powered / controlled using a Printed Circuit Board (PCB) mounted within the handle. The PCB may also interface with thermocouples contained within the ablation catheter. The PCB may also include an Electrically Erasable Programmable Read-Only Memory (EEPROM) chip to store / log information relevant to the procedure. The PCT may communicate with the ablation console via the lead cable.

The grip portion 138 defines a grip axis (labelled Z) in Figures 2a to 3 around which the user grips the handle 114. As can be seen in Figures 2a to 3, the grip axis Z may form a central longitudinal axis along the centre line of the portion of the housing which is gripped by the user. **In** the embodiment of Figures 2a to 6 the grip axis Z coincides with the lead cable connection axis X. That may not however be the case for all embodiments as will be described later. The grip axis may more generally be parallel to the lead cable and working channel connection axis X, Y. The grip axis Z is offset relative to the working channel connection axis Y as can be seen in Figures 2a to 3 such that they do not coincide. The grip portion 138 is not therefore concentric or in line with the working channel axis Y. This may help to provide a comfortable grip and improved weight balance of the handle. The separation in the grip axis Z and the working channel connection axis Y may create a pivot point between the handle and the delivery system so that the handle can be more easily manipulated.

A cross section through the grip portion 138 of the housing 126 through the plane marked AA in Figure 2a is shown in Figure 2b. The grip portion has a non-circular cross section. More specifically, the grip portion comprises two flat portions 139a, 139b, each extending longitudinally along the length of the grip portion 138. The flat portions 139a, 139b are provided on opposing sides of the grip portion 138 as shown in the Figures. The flat portions 139a, 139b are linked by curved portions 139c, 139d. The cross section therefore has a generally elongate shape (e.g. having a minor axis M which is shorter than a perpendicular major axis N). **In** other embodiments, the grip portion may have another elongate shaped cross section, such as an oval or elliptical cross section.

The cross-sectional shape of the grip portion facilitates grip in the desired orientation by not being perfectly cylindrical or round. The flat portions may sit into the palm of the hand when being gripped and the curved portions may accommodate the purlicue at one end of the grip portion and the wrapping of fingers on the other, in the correct (desired) orientation. This helps to guide the user to grip the handle in the correct orientation.

The major and minor axis of the grip portion cross section may be orientated with respect to the rest of the handle housing to give the desired grip orientation. The minor axis of the grip portion may be parallel with the plane in which the grip axis Z and working channel connection axis Y are arranged. More specifically, the minor axis may be arranged in the same plane as the grip axis Z and working channel connection axis Y as can be seen in Figure 3. This may orient the user's hand in the desired orientation.

Further details of the cross-sectional geometry of the grip portion 138 are shown in Figures 2c-2f. The flat portions 139a,b and the curved portions 139c,d of the shape of the cross section of the grip portion are labelled in Figure 2c only to aid clarity, but are also visible in Figures 2d-2f. The geometry of the cross-sectional shape of the grip portion 138 is defined by two overlapping ellipses and two tangential lines linking them, as can be seen in each of Figures 2c-f.

The centres of the ellipses are offset relative to each other by a distance A marked in Figure 2c. The centres of the ellipses are equally spaced from the grip axis Z so that the grip axis runs along the centreline of the grip portion 138. The offset of the centres of the ellipses results in the cross section of the grip being greater in length along its major axis compared to its minor axis as described above. The length across the major axis is labelled C in Figure 2d and the length across the minor axis is labelled B. Length C is greater than length B to give the non-circular profile of the grip portion. The flat portions 139a, 139b are defined by a respective tangential straight edge each joining one ellipse to the other as can be seen in Figure 2e (particularly in the close-up). The tangential lines defining the flat portions are parallel to the major axis of the grip portion. Figure 2f illustrates the offset mounting of the grip portion 138 relative to the working channel connection axis Y. The offset distance D between the grip axis and working channel connection axis is perpendicular to the centre or midpoint of the offset between the two ellipses, this dictates the centre point of the mounting portion of the handle.

The geometry of the cross section shown in the figures is to be understood one preferred embodiment, with other shapes being possible including a circular cross section.

In some embodiments, the grip portion may comprise a guide surface arranged to indicate the part of the handle to be gripped by the user. The guide surface may extend over some or all of the grip portion 138. The guide surface may be formed by part of the housing having a different textured finish or may be formed by a recessed region of the surface. This may guide the user to grip the handle in the correct orientation.

The handle 114 may be rotatable around the working channel connection axis during use. A rotatable coupling may be provided between the working channel 110 and the housing (e.g. a rotatable Luer connection) or the working channel connection interface 130 may comprise a component rotatably mounted to the housing 126 which couples to the working channel 110. By allowing relative rotation between the handle 114 and working channel 110 the user can rotate the handle 114 around the working channel connection axis Y so that it is held in a comfortable position. Moreover, as shown in Figures 5 and 6 this may allow the handle to be held comfortably in either the left or right hand without having to provide different left and right handed devices for different users.

In the presently described embodiment, the lead cable 116 extends along the length of the grip portion 136 of the handle 114 to provide structural strength as described above. In the presently described embodiment this is achieved by the lead cable connection axis X coinciding with the grip axis Z. The user therefore effectively grips around the lead cable 116 by holding the grip portion138 of the handle 114.

Referring again to Figures 2a and 4, the housing 126 comprises a recessed portion 140 in which the working channel 110 is located when connected. The recessed portion 140 comprises a reduced width portion of the length of the handle housing 126 and in the present embodiment provides a location for the working channel connection interface 130. The recessed portion 140 may provide space for the connection of the delivery system alongside the grip portion 126 of the handle 114 as can be seen in Figures 5 and 6. The recessed portion 140 may advantageously provide space for a variety of differently shaped delivery systems which may have a variety of different shaped components at the proximal end of the working channel. This may allow a variety of different delivery systems to be connected to the handle so that it can be used with a range of existing systems.

Figure 7 shows a cross section through another embodiment of a handle 114 of the present application. The handle 114 shown in Figure 7 includes components corresponding to those of the handle 114 shown in Figures 2 to 6 which are labelled accordingly with the same reference numerals.

The handle 114 of Figure 7 differs from the embodiment of Figures 2a to 6 by the inclusion of a sliding mechanism 142 via which the point of connection of the working channel 110 may slide relative to the housing 126 of the handle 114. In the embodiment of Figure 7 the handle 114 comprises a sliding component (e.g. carriage) 142a on which the working channel connection interface is located. The sliding component 142 is arranged to slide relative to the housing 126 along a track 142b. The sliding component 142a allows independent movement of the working channel 110 relative to housing 126, and therefore also relative to the catheter 110 when it is held fixed relative to the housing 126 (e.g. held manually by the user or more preferably using the catheter locking mechanism 136). This may allow the unsheathing of the ablation probe 112 from within the extended working channel 110 while maintaining the position of the distal end of the ablation probe 112 at the ablation site. For example, the ablation probe 112 can be positioned at the desired location while it is still within the extended working channel 110. Once in position, the ablation probe 112 can be locked in position relative to the handle 114 before the sliding mechanism 142 is operated to withdraw the working channel 110 relive to the handle 114 so that the distal tip of the ablation probe 112 extends from the working channel 110 while remining in a fixed position.

Another embodiment of a handle 214 for an ablation probe is illustrated in Figures 8 to 10. The handle 214 can be used with the ablation system 100 and delivery system 102 illustrated in Figure 1. Figure 8 shows a side view corresponding to Figure 2a, and Figures 9 and 10 show cross sectional views corresponding to Figure 4 in two different configurations. The handle 214 includes components corresponding to those of the embodiments described above with corresponding reference numerals used accordingly. Anything described in connection with other embodiments herein can be used in combination with that of Figures 8 to 10, and vice versa, and so will not be descried again.

The handle 214 shown in Figures 8 to 10 comprises a housing 226 that includes two parts - a first part 226a and a second part 226b. The first and second parts 226a, 226b are movable (e.g. slidable) relative to each other as shown in Figures 9 and 10. The first part of the housing 226 includes a lead cable connection interface 228. The second part of the housing comprises a working channel connection interface 230. The connection interfaces 228, 230 may be the same as those described in connection with Figures 2a to 4 and have connection axes arranged in a similar configuration e.g. with offset connection axes and the lead cable and working channel extending distally away from the housing as shown in the figures.

The ablation probe is secured within the first part of the housing 226a. As shown in Figures 9 and 10, the catheter 222 of the ablation probe is fixed to the manifold 232, which is in turn coupled to the lead cable 216 in a similar manner to as described above. The lead cable 216 and the catheter 222 are therefore fixed relative to the first part of the housing 226a. The second part of the housing 226b is arranged to provide a sliding connection to the catheter 222 such that the catheter can side relative to it. The catheter 222 is arranged to extend through the second housing 226b and exit the second housing 226b at a point that coincides with the working channel connection interface 230 so that it extends along the working channel similarly to the other embodiments described herein.

The second part of the housing 226b in the present embodiment comprises a travel column 226c along which the first part of the housing 226a may slide (translate) relative to the second part of the housing 226b. Other arrangements or mechanisms may be used to provide a relative sliding movement between parts of the housing 226.

By movement of the first part of the housing 226a with respect to the second part of the housing 226b the ablation probe (i.e. the catheter 222) may be moved relative to the working channel 210. This can be seen in Figures 9 and 10 in which the first part of the housing 226a is pushed downwards in the direction of the arrow in Figure 10 while the second part of the housing 226b remains stationary such that the catheter 222 is pushed along the working channel 210. This therefore provides similar movement of the catheter 222 as that provided by adjustment of the exposed loop portion 122a of the embodiment shown in Figures 2a to 6.

Referring again to Figure 8 the housing 226 comprises a grip portion 238 which may be gripped in a similar fashion to that described above and as shown in Figure **11** (and may have a similar shape). The grip portion 238 is provided on the first part of housing 226a. The second part of the housing 226b may therefore be held stationary (via its connection with the working channel 210) and the first part of the housing 226a translated relative to the second part of the housing to move the catheter 222 along the working channel 210.

The grip portion 238 of the first part of the housing 226a defines a grip axis Z similarly to the grip portion 138 described above. The grip axis Z in this embodiment extends long the central longitudinal axis of the first part of the housing 226a. As can be seen in Figure 11, the grip axis Z is offset relative to the connection axis Y of the working channel (and offset relative to the axis along which the catheter extends). This provides a similar offset grip location away from the axis of the working channel as described above. The grip axis Y may also be offset relative to the connection axis of the catheter 216 as shown in Figure 11, or may coincide with it.

Referring again to Figure 8, the housing 226 defines a recessed portion 240 similarly to the recessed portion 140 of housing shown in Figure 2a. In this embodiment however, the recessed portion 240 provides a location for the lead cable connection interface 228. The lead cable 216 extends along the length of the handle 214 similarly to as described above in connection with Figures 2a to 6 in order to similarly create a "spine" of the handle. In the embodiment of Figures 8 to **11** the lead cable however extends along the recessed portion 240 outside of the housing 226 rather than within the housing as is the case in Figure 4. A similar effect is however still achieved.

Referring again to Figure 1, in any of the embodiments described herein, the handle 114, 214 may form part of a handle system which further includes a lead cable-to-working channel connector 300. The connector 300 is arranged to connect between the lead cable 116, 216 and the working channel 110, 210 at a point along the lead cable 116, 216 spaced apart from the housing 126, 226 of the handle 114, 214. This may provide a secondary mounting point on the lead cable to which it is coupled to the delivery system. This may help provide further structural support by using the strength of the lead cable to support the working channel. It may also help to reduce the lever effect on the connection between the handle 114, 214 and the working channel 110, 210.

Figures 12 and 13 show further details of a manifold 332 which may be used to provide an electrical and fluidic connection between the lead cable and the catheter of the ablation probe. As described above, the ablation probe of the present application comprises an elongated flexible catheter 322, which comprises a catheter tube or shaft 350 in which the feed cable 352 is located. The catheter further comprises a coolant tube 354 which is arranged to carry a flow of coolant along the length of the catheter 322 to the applicator at its distal tip (not shown in Figures 12 and 13). A return flow of coolant flows along the space within the catheter shaft 350 (which forms a coolant return channel) to return coolant back to the proximal end of the ablation probe.

The manifold 332 may comprise an RF connector 356 arranged to provide an electrical connection between the feed cable 352 and the lead cable. The RF connector 356 may comprise a coaxial connector (or MCX connector (micro coaxial connector)). The manifold may further comprise a seal 358 (such as an O-ring seal) arranged to form a seal between the RF connector 356 and a housing 332a or other component(s) of the manifold.

The manifold 332 further comprises a coolant inflow channel 360 which is fluidly connected to the coolant tube 354 of the catheter 322, and which is fluidly connected to a coolant channel in the lead cable to provide a fluidic connection between them. The manifold 332 further comprises a coolant outflow channel 362 which is fluidly connected to the coolant return channel of the catheter 322, and which is also fluidly connected to a coolant channel in the lead cable to provide a fluidic connection between them. This allows coolant to be returned to the ablation console. The inflow and outflow coolant channels 360, 362 may be located with one on either side of the RF connector 356 as shown in Figure 12.

The manifold 332 shown in Figure 12 may have a minimal overall length L. This is advantageous in reducing the overall length of the feed cable contained within the catheter. Although the ablation probe must have sufficient length to reach the ablation site within the body, it is advantageous to reduce the feed cable length to minimise electrical losses in the system and maximise power delivery to the radiating tip and therefore the size of the ablation zone that can be achieved.

The coolant inflow channel 360 and coolant outflow channel 362 may be angled outwards relative to the housing (or centreline) of the manifold as shown in Figure 12. In an alternative embodiment shown in Figure 13, the coolant inflow channel 360 and coolant outflow channel 362 may be parallel to the housing of the manifold e.g. parallel to the RF connector. This may allow the length of the manifold (and therefore the feed cable length) to be further reduced.

The manifold described above may be used in any of the embodiments of the handle 114, 214 disclosed herein. The manifold may also be used with other ablation probe handles, for example those without the offset arrangement of connection interfaces described herein (i.e. without the lead cable and working channel connection axes being parallel and offset relative to each other and the connection interfaces being arranged such that when the working channel and lead cable are connected to the handle the working channel extends from the housing in the same direction relative to the housing as the lead cable).

The handle and handle systems disclosed herein may be used with a variety of different delivery system and not just that shown in Figure 1. The working channel interface is therefore adapted more generally to connect to any suitable working channel along which the ablation probe can extend and which can be inserted into the patient's body to reach an ablation site. For example, there may not necessarily be a separate main working channel 106 and extended working channel 110 as shown in Figure 1. There may be a single working channel, or any other arrangement of working channels so long as one can be coupled to the handle.

Although the embodiments described above include a microwave ablation probe the present application may apply equally to other types of ablation probes. The ablation probe may be arranged to emit radio frequency radiation in order to ablate tissue, rather than radiation at a microwave frequency. The microwave ablation system 102 may therefore more generally be an ablation system arranged to caused heating of tissue.

Various modifications will be apparent to the skilled person without departing form the scope of the claims. Any feature disclosed in connection with one embodiment may be used in combination with the features of another embodiment.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. An ablation probe handle (114) for use with an ablation probe (112) being suitable for use with an internal anatomy access system, the ablation probe handle comprising:
a housing (126) adapted to be gripped by the user;
a lead cable connection interface (128) adapted for connection to a lead cable (116) for supplying a source of microwaves, the lead cable connection interface defining a lead cable connection axis;
an ablation probe (120) mounting adapted to mount a catheter of an ablation probe within the housing; and
a working channel connection interface (130) adapted for connection to a working channel (110) of an internal anatomy access system, wherein the catheter of the ablation probe is arranged to extend from the housing along the working channel when the working channel is connected to the handle, the working channel connection interface (130) defining a working channel connection axis,
wherein the lead cable and working channel connection axes are parallel and offset relative to each other, and
wherein the working channel connection interface (130) and the lead cable connection interface (128) are arranged such that, when the working channel and the lead cable are connected to the handle, the working channel extends from the housing in the same direction relative to the housing as the lead cable.

2. The ablation probe handle according to claim 1, wherein the ablation probe mounting is adapted to secure the catheter of the ablation probe within the housing such that an exposed portion of the length of the catheter is formed outside of the housing, the exposed portion being proximal along the length of the catheter relative to the point at which the catheter extends from the working channel connection interface, wherein the catheter is movable along the working channel by adjusting the length of the exposed portion outside of the housing.

3. The ablation probe handle according to any preceding claim, wherein:
the housing comprises a first part and a second part;
the lead cable connection interface is provided at the first part of the housing;
the ablation probe mounting is adapted to secure the catheter of the ablation probe relative to the first part of the housing and slidable secure the catheter relative to the second part of the housing;
the working channel connection interface is provided at the second part of the housing; and
the first part of the housing is slidable relative to the second part of the housing, whereby the catheter is slidable along the length the working channel.

4. The ablation probe handle according to any preceding claim, wherein the handle comprises a catheter locking mechanism movable between an unlocked condition in which the catheter is slidable relative to the housing and an unlocked condition in which the catheter cannot slide relative to the housing.

5. The ablation probe handle according to any preceding claim, wherein the handle comprises a sliding mechanism comprising a sliding component on which the working channel connection interface is located, the sliding component being slidable relative to the housing to allow independent movement of the working channel relative to the catheter.

6. The ablation probe handle according to any preceding claim, wherein the housing comprises an elongate grip portion adapted to be gripped single-handedly by the user, and optionally wherein the grip portion has a non-circular cross section, and preferably comprises a surface having two flat portions linked by two curved portions.

7. The ablation probe handle according to claim 6, wherein the handle comprises one or more controls, and wherein the one or more controls are spaced apart along the length of the housing from the grip portion, preferably in a proximal direction along the length of the housing.

8. The ablation probe handle according to claim 6 or claim 7, wherein the grip portion defines a grip axis around which the housing is gripped by the user, wherein the grip axis is offset relative to the working channel connection axis.

9. The ablation probe handle according to any preceding claim, wherein the working channel connection interface is adapted to form a rotatable coupling with the working channel, whereby the handle is rotatable about the working channel connection axis during use.

10. The ablation probe handle according to any preceding claim, wherein a portion of the length of the lead cable extends along part of the length of the handle, preferably within or alongside a portion of the housing.

11. The ablation probe handle according to any preceding claim, wherein the housing comprises a recessed portion in which the working channel or lead cable connection interface is located.

12. The ablation probe handle according to any preceding claim, further comprising a manifold adapted to connect to the lead cable and the catheter of the ablation probe and arranged to form an electrical connection between them when connected and optionally wherein the lead cable is arranged to carry a flow of coolant and the manifold is arranged to form a fluidic connection between the catheter and lead cable when connected.

13. The ablation probe handle according to claim 12, wherein the manifold comprises any one or more of:
a) an RF connector such as a coaxial connector arranged to provide an electrical connection between a feed cable contained within the catheter of the ablation probe and the lead cable;
b) a coolant inflow channel arranged to provide a supply of coolant through the catheter of the ablation probe from the lead cable; and
c) a coolant outflow channel arranged to carry a return flow of coolant from the catheter of the ablation probe to the lead cable.

14. A handle system comprising the ablation probe handle according to any preceding claim, the handle system further comprising a connector which is connectable between the lead cable and the working channel at a point along the lead cable spaced apart from the housing.

15. An ablation system comprising the handle or handle system of any preceding claim dependent directly or indirectly on claim 2, and an ablation probe mountable within the housing of the handle, wherein one or both of:
a) the ablation probe comprises a catheter having one or more transparent coolant conduits arranged to carry a flow of coolant, and wherein coolant is visible through the exposed portion of the catheter outside of the housing during use; and/or
b) the catheter of the ablation probe has reference markers arranged to indicate the position of the catheter within the working channel.

## Patentansprüche

1. Ablationssondengriff (114) zur Verwendung mit einer Anablationssonde (112), die zur Verwendung mit einem Zugangssystem für die interne Anatomie geeignet ist, der Ablationssondengriff Folgendes umfassend:
ein Gehäuse (126), das eingerichtet ist, um vom Benutzer gegriffen zu werden;
eine Leitungskabelverbindungsschnittstelle (128), die zur Verbindung mit einem Leitungskabel (116) eingerichtet ist, um eine Mikrowellenquelle zu versorgen, wobei die Leitungskabelverbindungsschnittstelle eine Leitungskabelverbindungsachse definiert;
eine Ablationssondenbefestigung (120), die eingerichtet ist, um einen Katheter einer Ablationssonde in dem Gehäuse zu befestigen; und
eine Arbeitskanalverbindungsschnittstelle (130), die zur Verbindung mit einem Arbeitskanal (110) eines Zugangssystems für die interne Anatomie eingerichtet ist, wobei der Katheter der Ablationssonde eingerichtet ist, um sich von dem Gehäuse entlang des Arbeitskanals zu erstrecken, wenn der Arbeitskanal mit dem Griff verbunden ist, wobei die Arbeitskanalverbindungsschnittstelle (130) eine Arbeitskanalverbindungsachse definiert,
wobei die Leitungskabelverbindungsachse und die Arbeitskanalverbindungsachse parallel und versetzt zueinander sind, und
wobei die Arbeitskanalverbindungsschnittstelle (130) und die Leitungskabelverbindungsschnittstelle (128) so eingerichtet sind, dass, wenn der Arbeitskanal und das Leitungskabel mit dem Griff verbunden sind, der Arbeitskanal sich aus dem Gehäuse in die gleiche Richtung relativ zu dem Gehäuse erstreckt wie das Leitungskabel.

2. Ablationssondengriff nach Anspruch 1, wobei die Ablationssondenbefestigung eingerichtet ist, um den Katheter der Ablationssonde in dem Gehäuse derartig zu sichern, dass ein freiliegender Abschnitt der Länge des Katheters außerhalb des Gehäuses ausgebildet wird, wobei der freiliegende Abschnitt entlang der Länge des Katheters relativ zu dem Punkt proximal ist, an dem sich der Katheter aus der Arbeitskanalverbindungsschnittstelle erstreckt, wobei der Katheter entlang des Arbeitskanals durch Einstellen der Länge des freiliegenden Abschnitts außerhalb des Gehäuses beweglich ist.

3. Ablationssondengriff nach einem vorhergehenden Anspruch, wobei:
das Gehäuse einen ersten Teil und einen zweiten Teil umfasst;
die Leitungskabelverbindungsschnittstelle an dem ersten Teil des Gehäuses bereitgestellt ist;
die Ablationssondenbefestigung eingerichtet ist, um den Katheter der Ablationssonde relativ zu dem ersten Teil des Gehäuses zu sichern und den Katheter relativ zu dem zweiten Teil des Gehäuses verschiebbar zu sichern;
die Arbeitskanalverbindungsschnittstelle an dem zweiten Teil des Gehäuses bereitgestellt ist; und
der erste Teil des Gehäuses relativ zu dem zweiten Teil des Gehäuses verschiebbar ist, wodurch der Katheter entlang der Länge des Arbeitskanals verschiebbar ist.

4. Ablationssondengriff nach einem vorhergehenden Anspruch, wobei der Griff einen Katheterverriegelungsmechanismus umfasst, der zwischen einem entriegelten Zustand, in dem der Katheter relativ zu dem Gehäuse verschiebbar ist, und einem entriegelten Zustand beweglich ist, in dem der Katheter relativ zu dem Gehäuse nicht verschiebbar ist.

5. Ablationssondengriff nach einem vorhergehenden Anspruch, wobei der Griff einen Gleitmechanismus umfasst, der eine Gleitkomponente umfasst, auf der die Arbeitskanalverbindungsschnittstelle angeordnet ist, wobei die Gleitkomponente relativ zu dem Gehäuse verschiebbar ist, um eine unabhängige Bewegung des Arbeitskanals relativ zu dem Katheter zu ermöglichen.

6. Ablationssondengriff nach einem vorhergehenden Anspruch, wobei das Gehäuse einen länglichen Griffabschnitt umfasst, der eingerichtet ist, um vom Benutzer mit einer Hand gegriffen zu werden, und wobei der Griffabschnitt gegebenenfalls einen nicht kreisförmigen Querschnitt aufweist und vorzugsweise eine Oberfläche umfasst, die zwei flache Abschnitte aufweist, die durch zwei gekrümmte Abschnitte verbunden sind.

7. Ablationssondengriff nach Anspruch 6, wobei der Griff mindestens ein Bedienelement umfasst und wobei das mindestens eine Bedienelement entlang der Länge des Gehäuses von dem Griffabschnitt beabstandet ist, vorzugsweise in einer proximalen Richtung entlang der Länge des Gehäuses.

8. Ablationssondengriff nach Anspruch 6 oder 7, wobei der Griffabschnitt eine Griffachse definiert, um die herum das Gehäuse vom Benutzer gegriffen wird, wobei die Griffachse relativ zu der Arbeitskanalverbindungsachse versetzt ist.

9. Ablationssondengriff nach einem vorhergehenden Anspruch, wobei die Arbeitskanalschnittstelle eingerichtet ist, um eine drehbare Kupplung mit dem Arbeitskanal auszubilden, wodurch der Griff bei Verwendung um die Arbeitskanalverbindungsachse drehbar ist.

10. Ablationssondengriff nach einem vorhergehenden Anspruch, wobei sich ein Abschnitt der Länge des Leitungskabels entlang eines Abschnitts der Länge des Griffs erstreckt, vorzugsweise in oder entlang eines Abschnitts des Gehäuses.

11. Ablationssondengriff nach einem vorhergehenden Anspruch, wobei das Gehäuse einen vertieften Abschnitt umfasst, in dem die Arbeitskanalverbindungsschnittstelle oder die Leitungskabelverbindungsschnittstelle angeordnet ist.

12. Ablationssondengriff nach einem vorhergehenden Anspruch, weiterhin umfassend einen Verteiler, der eingerichtet ist, um mit dem Leitungskabel und dem Katheter der Ablationssonde verbunden zu werden, und eingerichtet ist, um eine elektrische Verbindung zwischen ihnen auszubilden, wenn er verbunden ist, und wobei gegebenenfalls das Leitungskabel eingerichtet ist, um einen Kühlmittelstrom zu führen, und der Verteiler eingerichtet ist, um eine fluidische Verbindung zwischen dem Katheter und dem Leitungskabel auszubilden, wenn er verbunden ist.

13. Ablationssondengriff nach Anspruch 12, wobei der Verteiler mindestens eines des Folgenden umfasst:
a) einen HF-Verbinder, wie beispielsweise einen Koaxialverbinder, der eingerichtet ist, um eine elektrische Verbindung zwischen einem Zuführungskabel, das in dem Katheter der Ablationssonde enthalten ist, und dem Leitungskabel bereitzustellen;
b) einen Kühlmittelzulaufkanal, der eingerichtet ist, um eine Zufuhr von Kühlmittel durch den Katheter der Ablationssonde aus dem Leitungskabel bereitzustellen; und
c) einen Kühlmittelauslaufkanal, der eingerichtet ist, um einen Kühlmittelrückfluss aus dem Katheter der Ablationssonde zu dem Leitungskabel zu führen.

14. Griffsystem, das den Ablationssondengriff nach einem vorhergehenden Anspruch umfasst, wobei das Griffsystem weiterhin einen Verbinder umfasst, der zwischen dem Leitungskabel und dem Arbeitskanal an einem Punkt entlang des Leitungskabels verbunden werden kann, der von dem Gehäuse beabstandet ist.

15. Ablationssystem, umfassend den Griff oder das Griffsystem nach einem vorhergehenden Anspruch, der unmittelbar oder mittelbar von Anspruch 2 abhängig ist, und eine Ablationssonde, die in dem Gehäuse des Griffs befestigt werden kann, wobei:
a) die Ablationssonde einen Katheter umfasst, der mindestens eine transparente Kühlmittelleitung aufweist, die eingerichtet ist, um einen Kühlmittelstrom zu führen, und wobei bei Verwendung Kühlmittel durch den freiliegenden Abschnitt des Katheters außerhalb des Gehäuses sichtbar ist; und/oder
b) der Katheter der Ablationssonde Referenzmarkierungen aufweist, die eingerichtet sind, um die Position des Katheters in dem Arbeitskanal anzugeben.

## Revendications

1. Manche de sonde d'ablation (114) destiné à être utilisé avec une sonde d'ablation (112) adaptée pour une utilisation avec un système d'accès à l'anatomie interne, le manche de sonde d'ablation comprenant :
un boîtier (126) adapté pour être saisi par l'utilisateur ;
une interface de connexion de câble conducteur (128) adaptée pour la connexion à un câble conducteur (116) pour fournir une source de micro-ondes, l'interface de connexion de câble conducteur définissant un axe de connexion de câble conducteur ;
un montage de sonde d'ablation (120) adapté pour monter un cathéter d'une sonde d'ablation à l'intérieur du boîtier ; et
une interface de connexion de canal de travail (130) adaptée pour la connexion à un canal de travail (110) d'un système d'accès à l'anatomie interne, le cathéter de la sonde d'ablation étant agencé pour s'étendre depuis le boîtier le long du canal de travail lorsque le canal de travail est connecté au manche, l'interface de connexion de canal de travail (130) définissant un axe de connexion de canal de travail,
les axes de connexion de câble conducteur et de canal de travail étant parallèles et décalés l'un par rapport à l'autre, et
l'interface de connexion de canal de travail (130) et l'interface de connexion de câble conducteur (128) étant agencées de telle sorte que, lorsque le canal de travail et le câble conducteur sont connectés au manche, le canal de travail s'étend depuis le boîtier dans la même direction par rapport au boîtier que le câble conducteur.

2. Manche de sonde d'ablation selon la revendication 1, le montage de sonde d'ablation étant adapté pour fixer le cathéter de la sonde d'ablation à l'intérieur du boîtier de telle sorte qu'une partie exposée de la longueur du cathéter soit formée à l'extérieur du boîtier, la partie exposée étant proximale le long de la longueur du cathéter par rapport au point auquel le cathéter s'étend depuis l'interface de connexion de canal de travail, le cathéter étant mobile le long du canal de travail en ajustant la longueur de la partie exposée à l'extérieur du boîtier.

3. Manche de sonde d'ablation selon l'une quelconque des revendications précédentes :
le boîtier comprenant une première partie et une seconde partie ;
l'interface de connexion de câble conducteur étant fournie au niveau de la première partie du boîtier ;
le montage de sonde d'ablation étant adapté pour fixer le cathéter de la sonde d'ablation par rapport à la première partie du boîtier et fixer de manière coulissante le cathéter par rapport à la seconde partie du boîtier ;
l'interface de connexion de canal de travail étant fournie au niveau de la seconde partie du boîtier ; et
la première partie du boîtier étant coulissante par rapport à la seconde partie du boîtier, de sorte que le cathéter est coulissant le long de la longueur du canal de travail.

4. Manche de sonde d'ablation selon l'une quelconque des revendications précédentes, le manche comprenant un mécanisme de verrouillage de cathéter mobile entre une condition déverrouillée dans laquelle le cathéter est coulissant par rapport au boîtier et une condition déverrouillée dans laquelle le cathéter ne peut pas coulisser par rapport au boîtier.

5. Manche de sonde d'ablation selon l'une quelconque des revendications précédentes, le manche comprenant un mécanisme de coulissement comprenant un composant coulissant sur lequel l'interface de connexion de canal de travail est située, le composant coulissant étant coulissant par rapport au boîtier pour permettre un mouvement indépendant du canal de travail par rapport au cathéter.

6. Manche de sonde d'ablation selon l'une quelconque des revendications précédentes, le boîtier comprenant une partie de préhension allongée adaptée pour être saisie d'une seule main par l'utilisateur, et éventuellement la partie de préhension ayant une section transversale non circulaire, et de préférence comprenant une surface ayant deux parties plates reliées par deux parties incurvées.

7. Manche de sonde d'ablation selon la revendication 6, le manche comprenant une ou plusieurs commandes, et la ou les commandes étant espacées le long de la longueur du boîtier depuis la partie de préhension, de préférence dans une direction proximale le long de la longueur du boîtier.

8. Manche de sonde d'ablation selon la revendication 6 ou la revendication 7, la partie de préhension définissant un axe de préhension autour duquel le boîtier est saisi par l'utilisateur, l'axe de préhension étant décalé par rapport à l'axe de connexion de canal de travail.

9. Manche de sonde d'ablation selon l'une quelconque des revendications précédentes, l'interface de canal de travail étant adaptée pour former un couplage rotatif avec le canal de travail, de sorte que le manche est rotatif autour de l'axe de connexion de canal de travail pendant l'utilisation.

10. Manche de sonde d'ablation selon l'une quelconque des revendications précédentes, une partie de la longueur du câble conducteur s'étendant le long d'une partie de la longueur du manche, de préférence à l'intérieur ou le long d'une partie du boîtier.

11. Manche de sonde d'ablation selon l'une quelconque des revendications précédentes, le boîtier comprenant une partie en retrait dans laquelle l'interface de connexion de canal de travail ou de câble conducteur est située.

12. Manche de sonde d'ablation selon l'une quelconque des revendications précédentes, comprenant en outre un collecteur adapté pour se connecter au câble conducteur et au cathéter de la sonde d'ablation et agencé pour former une connexion électrique entre eux lorsqu'ils sont connectés et éventuellement le câble conducteur étant agencé pour transporter un flux de liquide de refroidissement et le collecteur étant agencé pour former une connexion fluidique entre le cathéter et le câble conducteur lorsqu'ils sont connectés.

13. Manche de sonde d'ablation selon la revendication 12, le collecteur comprenant un ou plusieurs des éléments suivants :
a) un connecteur RF tel qu'un connecteur coaxial agencé pour fournir une connexion électrique entre un câble d'alimentation contenu dans le cathéter de la sonde d'ablation et le câble conducteur ;
b) un canal d'entrée de liquide de refroidissement agencé pour fournir une alimentation en liquide de refroidissement à travers le cathéter de la sonde d'ablation depuis le câble conducteur ; et
c) un canal de sortie de liquide de refroidissement agencé pour transporter un flux de retour de liquide de refroidissement depuis le cathéter de la sonde d'ablation vers le câble conducteur.

14. Système de manche comprenant le manche de sonde d'ablation selon l'une quelconque des revendications précédentes, le système de manche comprenant en outre un connecteur qui est connectable entre le câble conducteur et le canal de travail à un point le long du câble conducteur espacé du boîtier.

15. Système d'ablation comprenant le manche ou le système de manche selon l'une quelconque des revendications précédentes dépendant directement ou indirectement de la revendication **2,** et une sonde d'ablation montable à l'intérieur du boîtier du manche, avec l'un des éléments suivants ou les deux :
a) la sonde d'ablation comprenant un cathéter ayant un ou plusieurs conduits de liquide de refroidissement transparents agencés pour transporter un flux de liquide de refroidissement, et le liquide de refroidissement étant visible à travers la partie exposée du cathéter à l'extérieur du boîtier pendant l'utilisation ; et/ou
b) le cathéter de la sonde d'ablation ayant des marqueurs de référence agencés pour indiquer la position du cathéter à l'intérieur du canal de travail.
